Europäisches Patentamt

(19) European Patent Office

Office européen des brevets

(11) Publication number: **0 286 405**
**A2**

(12) # EUROPEAN PATENT APPLICATION

(21) Application number: **88303109.8**

(22) Date of filing: **07.04.88**

(51) Int. Cl.4: **C 07 K 3/18**
C 12 N 5/00, C 12 P 21/00,
C 12 Q 1/24
// A61K39/395, A61K39/00,
G01N33/569

(30) Priority: **08.04.87 US 36027**

(43) Date of publication of application:
**12.10.88 Bulletin 88/41**

(84) Designated Contracting States:
**AT BE CH DE ES FR GB GR IT LI LU NL SE**

(71) Applicant: **SYNBIOTICS CORPORATION**
**11011 Via Frontera**
**San Diego California 92127 (US)**

(72) Inventor: **Anderson, Darrel R.**
**1429 E.Magnolia**
**Escondido California 92027 (US)**

(74) Representative: **Harrison, David Christopher et al**
**MEWBURN ELLIS & CO 2/3 Cursitor Street**
**London EC4A 1BQ (GB)**

(54) **Method for the early selection of anti-idiotype-antibody internal images.**

(57) A method of selecting an anti-idiotype antibody having a high probability of having an internal image of a pathogenic antigen which comprises incubating a collection of antibody-producing cells obtained from a primary producer host that have been activated with an affinity purified antiserum specific for a pathogen with a labeled affinity purified selective antiserum specific for the pathogen and selecting immune cells that bind the detectably labeled antiserum.

EP 0 286 405 A2

Bundesdruckerei Berlin

## Description

## METHOD FOR THE EARLY SELECTION OF ANTI-IDIOTYPE-ANTIBODY INTERNAL IMAGES

This invention is related to techniques for the production of anti-idiotype antibodies useful as numerous products, such as vaccines.

There are two general types of vaccines: active and passive. Passive vaccines comprise antibodies administered to the host being protected that interact directly with a pathogenic organism and aid in the removal of the pathogen by the host's own immune system. Active vaccines are compositions that elicit an immune response, including the production of antibodies, when administered to a host being protected. The immune response causes the host to become resistant to the pathogen, typically a bacterium, virus, or other parasite.

Antigenic preparations used as active vaccines in the past have typically consisted of killed pathogens, live pathogens attenuated in some manner to reduce pathogenicity, and extracts from such organisms. More recently, anti-idiotypic antibodies have been proposed for use as vaccines. The anti-idiotypic antibodies are typically prepared by administering a first antibody raised against a pathogen, which is typically referred to as the idiotypic antibody, in order to produce antibodies against the idiotypic antibody; i.e., anti-idiotypic antibodies. At least some of the anti-idiotypic antibodies produced in this manner will have a binding site that reflects the steric and polar configuration of the original antigen from the pathogen, since at least some of the anti-idiotype antibodies will recognize the binding site on the idiotypic antibody that recognizes the antigen from the pathogen. Anti-idiotype antibodies having a surface that mimics the surface of the antigen from the pathogen are said to have internal images of the antigen. Such antibodies are useful as vaccines or otherwise as substitutes for the original antigen.

A report of polyclonal anti-idiotypic antibodies was published in 1982, based on antisera against three different idiotypic monoclonal antibodies specific for a pathogen. Monoclonal anti-idiotype antibodies utilized as a vaccine capable of regulating or at least participating in antibody response have since been reported.

However, there remains a problem in identifying anti-idiotype antibodies with internal images of antigens from pathogens. As pointed out in a review article published in 1984 by Hay etal. (referenced below), not all antibodies typically thought of as anti-idiotypes are useful as vaccines. The paratope, i.e., that part of the combining site of the idiotypic antibody that makes contact with the antigen, may or may not be the site recognized by the anti-idiotype antibody. If the site is not the same, then the anti-idiotype antibody is unlikely to be useful as a vaccine since it is binding to a portion of the variable region of the idiotype different from the site that binds the antigen and therefore is not mimicking the antigen surface. In fact, when the large surface area of an antibody relative to the paratope that forms the binding site to the original antigen is considered, it is apparent that only a very few anti-idiotype antibodies will have an internal image of the pathogen and therefore be capable of functioning as an equivalent to the original antigen.

Prior art techniques have not provided a solution for this problem but have continued to prepare hybridomas from all antibody-producing B cells. Therefore, many non-useful fusions are expanded and later tested, resulting in considerable lost labor and expense. Accordingly, new techniques for the preparation of internal image anti-idiotype antibody products remained needed.

EARLIER PUBLICATIONS IN THIS FIELD

Hay, et al., "Idiotype Vaccines," in Immune Intervention, Academic Press, 1984, pages 117-138, describes a number of strategies and techniques for preparing vaccines based on anti-id antibodies. An early hypothesis involving the use of anti-idiotypic antibodies in a possible application to vaccine production is described in Nisonoff and Lamoyi, Clinical Immunology and Immunopathology (1981) 21:397-406. An anti-idiotypic antibody vaccine for type B viral hepatitis in chimpanzees is described in Kennedy et al., Science (April 1986) 212:220-223. Monoclonal internal image anti-idiotypic antibodies of hepatitis B surface antigen are described in Thanavala et al., Immunology (1985) 55:197-204. U.S. Patent No. 4,490,358 to Greene et al. describes the use of anti-idiotypic antibodies to block the bonding of infectious organisms to cell receptors.

The present invention provides a method of selecting internal image anti-idiotype antibodies and cells that produce them for use as antigen equivalents, for example in the production of a vaccine in a pathogen-susceptible host species. The process comprises (1) incubating a collection of antibody-producing cells obtained from a primary producer host, which have been activated with an affinity purified antiserum from a secondary producer host that has been immunized with an antigen from the pathogen, with a labeled, affinity purified, antiserum from a selective host that has been immunized with an antigen from the pathogen and (2) selecting antibody-producing cells that bind the labeled antiserum. The two affinity purified antisera are both prepared utilizing antigens from the pathogen against which protection is desired in order to select for antibodies that specifically bind to such antigens. The selected antibody-producing cells can be immortalized, for example by fusing with a continuous cell line to produce hybridomas that will in turn produce internal image anti-idiotype antibodies. The method may accordingly eliminate non-productive cell lines at an early stage in the process of identifying the characteristics of the anti-idiotype antibodies and therefore greatly reduces labor and material costs normally incurred during cell expansion and culture. Other advantages are identified in the following discussion of specific embodiments.

The present invention utilizes an affinity purified antiserum specific for one or more antigens of the pathogen against which protection is desired. This purified immune serum, typically obtained from the pathogen-susceptible host, is utilized for early detection of cells producing internal image, anti-idiotype antibodies. The anti-idiotype-antibody-producing cells (described herein as being obtained from a primary producer species to indicate that they are producers of the anti-idiotype antibodies) themselves are prepared by activation with a second affinity purified immune serum from a secondary producer host or secondary producer host cell line that has been challenged with the pathogen against which protection is desired, with an antigen derived from the pathogen, or with an equi valent (e.g., artificial) antigen. Using multiple polyclonal, affinity purified reagents has proven to be useful in eliminating culture of undesired non-internal-image-antibody-producing cells at an early stage of culture while maintaining a good selection of antibody-producing cells for use in the production of an anti-idiotype antibody having an internal image of the antigen which the anti-idiotype antibody is replacing.

There are a number of possible ways of implementing the selection process of the invention to achieve the same end result. However, two idiotypic antisera are required, with antisera from two different species of animal hosts being highly desirable. One species will typically be the pathogen-susceptible species for which protection is desired. A purified idiotypic antiserum from this host is desirable as it tends to select the anti-idiotype antibodies which the susceptible host is most likely to recognize and against which a strong immune response is most likely to develop. Since this antiserum is utilized to select the anti-idiotype-antibody-producing cells, the organism from which this antiserum is obtained is referred to as the selective host.

An affinity purified idiotypic antiserum (i.e., prepared by challenge with the antigen) is also required, desirably from a host species different from that of the selective host. Since this antiserum is utilized to produce the final anti-idiotype antiserum, the host species from which the antiserum is obtained is referred to as the secondary producer host. The secondary producer host is utilized initially to produce an idiotypic antiserum against the pathogen, an antigen therefrom, or its equivalent. This idiotypic antiserum is then utilized, either in the same or a different species of animal, to produce B cells that secrete anti-idiotype antibodies and other antibodies directed against the idiotype antibody. Antibody-secreting cells are then subjected to the selection process of the invention utilizing the affinity purified antiserum from the selective host as described herein.

The secondary producer host species can either be the same as or is preferably different from the species used to produce the anti-idiotype antibodies, referred to herein as primary producer species. Using interspecies immunization typically produces a strong immune response without requiring the use of adjuvants that are likely to increase the number of antibody-producing cells that secrete antibodies to antigens other than the idiotype antibody.

When interspecies challenge is used to produce B cells that will be selected for production of an internal image anti-idiotype antibody, it is useful to utilize a blocking step to prevent selection of cells that produce antibodies for interspecies homology regions of antibodies. This can be accomplished by preincubating the B cells with non-specific antiserum from the selector host.

The use of a selection species that is different from the second producer species is highly desirable to prevent two way capture during the internal image detection and selection process. If, for example, one used rabbit idiotype to induce mouse anti-idiotypes and then used the same antibodies from rabbit attached with a label for detection, there would be present in the mouse B-cell population a number of cells that contain surface antibodies which bear anti-rabbit specificities. Although they bear no internal image they could still capture fluorescent-labeled selection reagent, resulting in false positive labeling. The use of a separate species eliminates this two way recognition except for a very small number of cells bearing specificity to interspecies homology regions. These cells can be dealt with by preabsortion with non-labeled non-immune sera from the selection species, as outlined below.

The present method can be practised by utilizing any animal capable of producing antibodies as either the primary producer host, secondary producer host, or selective host species. Mammals and birds are particularly useful. Humans, rabbits, mice, rats, dog, cats, chickens, horses, cattle, hamsters, guinea pigs, and the like can be utilized as sources for the immune cells or host cells. Rabbits, mice, rats, and hamsters are particularly useful as sources of B cells for production of the anti-idiotype antibody and as sources of the affinity purified antiserum from the secondary producer host. Rabbits are especially useful as they provide large amounts of polyclonal antibodies and are easily maintained.

The pathogen against which protection is being sought can be any pathogen capable of eliciting an immune response. Examples include viruses, bacteria, and eukaryotic parasites, whether unicellular or multicellular. For example, a list of algae, protozoa, bacteria, and fungi having potential as infectious disease pathogens has been published by the U.S. Department of Health, Education and Welfare, Public Health Service, in a publication entitled "Classification of Etiologic Agents on the Basis of Hazard." The latest edition of this publication can be consulted for a list of pathogenic organisms. A similar listing is set forth in the American Type Culture Collection Catalog of Strains I. Again, the latest available edition should be consulted, but the 15th edition (1982) sets forth a list of potentially pathogenic agents on pages 13-18. Examples include Leishmania adleri, Plasmodium berghei, Haemophilus influenzae, Salmonella (all species), Candida bombi, Bacillus anthracis, Clostridium botulinum, Legionella (all species), Vibrio cholerae, Yersinia pestis, Entamoeba histolytica, Trypanosoma cruzi, Arboviruses, Rhabdoviruses, pasturella multocida, Pseudomonas mallei, Leishmania braziliensis, Plasmodium falciparum, Plasmodium knowlesi, adenoviruses (e.g., avian, type 6; bovine, type 4; and bovine, type 8), Japanese encephalitis, feline infectious peritonitis coronavirus, myxoviruses (e.g., Newcastle Disease), porcine enterovirus, and hog cholera togavirus.

Standard techniques for activating immune cells with antigens from pathogens can be utilized in the practice of the invention. Both in vivo and in vitro techniques of stimulating production of antibodies can be utilized. For example, animals can be infected with whole pathogens, with immunogenic preparations prepared therefrom, or with artificial antigens that mimic natural antigens from the pathogens. For example, when utilizing a pathogen with the potential of severely affecting the health of the animal being immunized, preparations of immunogenic components from the pathogen are preferred. Alternatively, an in vitro activation of spleen cells or other immune cells can be utilized with potentially dangerous pathogens.

Although the technique of the present method was designed for use in selecting antibody-producing cells that can be fused with immortal cells lines to produce hybridomas (or otherwise immortalized; e.g., by treatment with Epstein-Barr virus) and therefore produce anti-idiotype antibodies in large amounts, the method itself does not rely on producing hybridomas (or other immortalized cells) or monoclonal antibodies. Rather, the selection process provides antibody-producing cells that can be utilized in any immortalization, hybridoma, or antibody-producing procedures. Accordingly, the invention can be practiced with any known or later discovered technique for producing monoclonal antibodies.

The invention does utilize the technique of affinity purification of antiserum. The techniques of affinity purification are well known and need not be described here in detail. However, the following general description is provided to illustrate this aspect of the invention and to show how it fits into the overall process.

Affinity purification relies on the binding affinity between the two members of a specific binding pair. The specific binding reaction between the two binding-pair members is utilized to separate one of the two binding-pair member from an impure solution containing it. The second binding-pair member is immobilized on a solid support or is otherwise capable of removing the first binding-pair member from the impure solution. In the present case, the binding pair consists of an antibody and an antigen with which the antibody specifically binds. Since antibodies present in antisera are being purified in the present invention, the technique is most readily carried out by binding whole pathogens, parts of pathogens, or antigens from the pathogen to a solid surface. An antiserum produced against the same pathogen is then contacted with the immobilized antigens. Antibodies specific for particular antigens of the pathogen become bound to the immobilized antigens while non-specific antibodies or antibodies specific for other antigens pass by the immobilized antigens without being trapped. Bound antibodies are then separated from the immobilized antigens by various techniques, such as changing the ionic strength or pH of the medium or by utilizing an excess of the same antigens to compete with and remove the bound antibodies.

In order to increase surface area and therefore increase the amount of antigen that can be immobilized, affinity purification is often carried out in a chromatography column using a finely divided gel having antigen bound to its surfaces. Such materials are commercially available, and their composition is not material to the practice of the present invention.

There are a number of possible ways to implement the assay of the invention to achieve the same end results. A number of these techniques are illustrated below in examples of applying the method to canine heartworm disease. These examples are not intended to limit the present invention to canine heartworm disease vaccine production. Rather, the use of this particular pathogen in these examples is illustrative only, and other pathogens can be used in place of canine heartworms with equivalent results.

The desired end product in all cases is a B cell, selected by the use of this method, which produces antibodies bearing internal images of pathogen antigens without their hypervariable regions. The selected cells can then be used to prepare hybridomas or otherwise immortalized. The antibodies secreted by these immortalized cells are destined to be used as a vaccine against the pathogen, in these specific examples for protection of dogs against canine heartworm disease and to prevent spreading of the infestation by mosquitoes. The antibodies can also be utilized in diagnostic assays or for any other purpose for which an antigen equivalent is utilized.

In a first example of the technique, a dog that has been exposed to the heartworm pathogen and has mounted an immune response can be utilized as the source of the selective antiserum. This immune serum is affinity purified. The resulting affinity purified antiserum provides a source of polyclonal idiotypic antibodies that the host has produced, in this first instance by natural infection. This pool of antibodies recognizes a wide range of foreign antigens associated with the infective agent. It also represents a response to the infective agent determinants that the host animal will most likely respond to if given a vaccine. The affinity purified immune serum is highly sensitive to heartworm and is not cross-reactive with any other species of antibody. As will become clear below, this reagent is utilized in a selection step prior to the time of selecting specific antibodies in previous methods.

By injecting a heartworm immunogenic preparation into a rabbit (the secondary producer host), large amounts of polyclonal idiotypic antibodies against a large array of epitopes on the heartworm are again produced. These idiotypic antibodies can then be affinity purified and the resulting affinity purified antiserum injected into a different species, for example, mice, which serves as the primary producer species for generating anti-idiotypic antibodies. Use of a second species requires no modification to provide hyperimmune responses, since interspecies antibodies are being utilized. The hyperimmune spleen or other source of antibody-producing cells therefore contains a large popuation of B cells with internal-image-bearing antibodies present on their cell surfaces. These anti-idiotype-producing B cells are selected for in the next stage of the process utilizing the affinity purified selective antiserum described in the preceeding paragraph.

A label capable of producing a detectable signal is attached to the affinity purified selective antiserum in order that a binding reaction between the idiotypic antibodies present in this antiserum and the anti-idiotypic antibodies being produced by the producer B cells can be detected. Although any type of detectable marker can theoretically be utilized, fluorescent markers are preferred because of the ease with which cells can then be separated utilizing a fluorescent activated cell sorter (FACS).

Improved selection can be obtained by pre incubation of the spleen cell population from the primary producer species with non-immune, selective-host serum in order to block all spleen cells with surface antibodies specific for interspecies homology regions. The remaining unblocked B cells with surface antibodies specific for the hypervariable region of the idiotypic antibodies utilized to produce the immune response are available for reaction with the labeled affinity purified selective antiserum. Cells separated under these conditions are then used exclusively for later steps such as fusion with myeloma cells to produce hybridomas. The resulting hybrids should in theory all be producing anti-idiotype antibodies capable of themselves inducing idiotypic antibodies specific for an antigen present in the pathogen (i.e., they should have internal images of antigens present in the pathogen). Hybridomas that produce antibodies to other locations in the hypervariable region of the original idiotypic antibodies are eliminated.

A number of advantages and special features of the present method can be illustrated with the general procedure described above. The procedure reduces the labor and cost required in raising large numbers of hybridomas and then carrying out selection at a later stage, after production of hybridomas, as was typically done in the prior art.

Another advantage lies in the ease with which antibody preparations can be tailored for specific types of uses. For example, a number of parasites, including heartworms, undergo a series of stages within their life cycle. With heartworms, a dog is infected with larvae from a mosquito vector. The larvae migrate to the heart to mature before reproducing. If a dog is infected with larvae (such as with an attenuated larvae vaccine), B cells in the dog will respond to antigens found on the larvae. If some or all of these antigens disappear as the worm matures, the animals will not be adequately protected from adult or intermediary stages of maturing worms. The present invention allows selection of an affinity purified primary host antiserum that will produce a highly specific immunity, if desired. For example, an affinity column can be prepared utilizing both larval and adult male and female worm extracts.

This produces antibodies binding to the column that are directed against antigens found on both larval and adult heartworms of either sex. Furthermore, since male or female worms can be identified, it is also possible to select for sex-selective antibodies, thereby allowing detection of sex-specific anti-idiotypes. An anti-idiotype antibody product that elicits a sex-specific immune response would allow development of methods for interfering in reproduction and life cycles of numerous pests by inactivating or removing one or the other sexual partner.

If other antigens are of particular interest, specific antisera can be prepared simply by preparing an appropriate affinity absorbant that will select for antibodies binding with the desired antigen. By selecting the antigen utilized to affinity purify the idiotypic antiserum from the selective and/or secondary host, anti-idiotype antibodies having the desired characteristics can readily be selected. For example, vaccines specifically directed against larval infestation can be prepared as well as anti-idiotype antibodies reactive with a single species or strain and not with related organisms. While this latter type of anti-idiotype antibody might be less useful as a vaccine because of its narrow specificity, it would be useful in diagnostic techniques using anti-idiotype antibodies in place of actual antigens. Such diagnostic techniques are useful in that they avoid the necessity of purifying antigen but instead rely on the production of large amounts of the anti-idiotype antibody by hybridomas for use as an equivalent material.

A particular advantage of the present invention that became apparent during its development was that the antibodies that produced the greatest response as internal images often have very low or marginal responses to the immunizing secondary host antibody. Previous methods tended to utilize only the stronger positive responders to the immunizing antibody as a criteron for selection. This suggests that previous methods may have resulted in overlooking or selecting against those cell lines that were in fact producing the most desirable products since there does not appear to be a correlation between a strong positive responder cell line and a cell line that in fact has the best internal image for use as a vaccine.

A number of alternative methods of practicing the invention can be utilized. Some of these have specific advantages and are therefore particularly suitable for use in certain cases. For example, certain pathogens are highly fatal to their principal host. In many such cases it will be more feasible to use a different host as the selective host rather than using attenuated pathogens, which may either be not readily available or may be so modified as to not produce the desired kind of immune response, therefore limiting the usefulness of any anti-idiotype vaccine that might be produced from the attenuated organisms. An example of this situation is feline infectious peritonitis coronavirus, which is highly fatal to infected cats and is not readily available in attenuated form. Rabbits or goats can be utilized as the selective host for developing a vaccine to be utilized in cats.

One example of an alternative technique for use when the selective host animal is at great danger from the pathogen is the following. Instead of using a principal host of the parasite as the selective host species for production of the labeled antiserum, a different animal species is utilized. To continue with the heartworm example, a rabbit can be utilized as a selective host for treatment with an immunogenic extract from adult and female heartworms. The rabbit produces an anti-heartworm antiserum which is then purified on a Sepharose-heartworm column to give an affinity purified rabbit anti-heartworm antiserum. This primary purified antiserum is then labeled (for example, with FITC) and utilized in the labeling and selection step.

Concurrently or at a different time, a secondary producer host is treated with an immunogen to produce an initial anti-heartworm antiserum. This secondary host can either be the principal host of the pathogen (a dog in this case) or it may be an entirely different species (e.g., a hamster). The anti-heartworm antiserum from the secondary host is also affinity purified on the same or a different Sepharose-heartworm column to give an affinity purified secondary host anti-heartworm antiserum. This is the idiotypic antiserum used to activate B cells from the primary producer species that will eventually be fused with myeloma cells to produce anti-idiotype antibody for use in vaccines. The primary producer species is preferably a different species as described above in order to maximize immune response. The primary producer species may be, for example, a mouse. Hyperimmune mouse spleen cells or other B cells are then preincubated with non-immune-affinity-absorbed, non-fluorescent rabbit serum to block spleen cells specific for interspecies homology regions. The resulting blocked cells are then incubated with fluorescent-labeled rabbit affinity purified anti-heartworm antiserum, prepared as described above. The resulting spleen cells that fluoresce are separated by a fluorescence-activated cell sorter that selects fluorescent-labeled spleen cells. These spleen cells can then be hybridized using standard techniques to produce an anti-idiotype antibody that almost certainly has an internal image of the antigen from the pathogen against which protection is desired.

Another alternative technique utilizes a single species of animal as both the secondary and primary producer hosts. Again illustrating the technique with canine heartworms, a selective host (dog) is immunized with a heartworm preparation as described above. The dog anti-heartworm antiserum is then affinity purified on a Sepharose-heartworm column to produce an affinity-purified dog anti-heartworm antiserum which is then labeled (for example, with FITC) to produce a fluorescent-labeled dog antiserum. Concurrently, a secondary producer host (e.g., mouse) is treated with an immunogenic preparation from the same pathogen (which may be the same as or different from the immunogenic preparation utilized to immunize the selective host, but is preferably capable of producing a broad immune response). A secondary host (e.g., mouse) anti-heartworm antiserum is thus obtained. This idiotypic antiserum is affinity purified on a Sepharose-heartworm column to produce an affinity purified mouse anti-heartworm antiserum. Since in this example of techniques an intraspecies immunization is utilized, the idiotypic mouse antibody is preferably administered with an agent capable of increasing the immune response, such as KLH. The resulting hyperimmune mouse spleen cells are then incubated with the fluorescent-labeled idiotypic antibody, and the resulting fluorescent-labeled cells are sorted utilizing FACS techniques. The selected cells can then be expanded to produce monoclonal or polyclonal anti-idiotype-antibody-producing hybridoma cells lines.

In other instances it may not be feasible or desirable to employ fluorescent cell sorting techniques. The primary host affinity-purified antiserum can nonetheless be utilized in conjunction with other reagents covalently linked with peroxidase, biotin, or any other detectable means. The detection process can even be carried out at the post-fusion stage for selecting any positive hybrids that are to be expanded for use in preparing vaccines.

The invention now being generally described, the same will be better understood by reference to the following examples which are included for purposes of illustration only and are not intended to be limiting of the invention unless so specified.

EXAMPLE

A number of anti-idiotype-producing hybridomas were previously produced utilizing a single heartworm-specific antibody (D.I. 130.1) as the idiotype. Using the prior art technique of selecting producer cell lines reactive with the immunizing antibody, a total of 324 hybridoma cell lines positive for D.I. 130.1 were detected in a standard post-fusion screening assay. By utilizing an affinity-purified dog anti-heartworm antiserum, purified against a chromatography column containing immobilized heartworm antigens (i.e, a selective antiserum utilized in a process of the present invention), it was determined that only 39 of the 324 hybrids were secreting internal image anti-idiotypes. Thus, 90% of the hybrids selected by the prior art process were of no use as anti-idiotype vaccines. Since about 20 cells lines is the maximum normally expanded in any one experiment, use of the affinity purified antiserum as described not only reduced the labor by approximately 90%, it also allowed expansion of all likely cell lines in two experiments. Given normal time constraints, it is unlikely that all 324 of the initially selected cell lines (selected by the prior art process) could have been maintained over the time necessary to carry out 16 or 17 expansion and characterization experiments. Thus, it is likely that a number of potentially useful vaccines would have been lost using prior art techniques.

**Claims**

1. A method of selecting an anti-idiotype-antibody-producing cell having an internal image for an antigen of a pathogen, which comprises:

incubating a collection of antibody-producing immune cells of a primary producer host or antibody-producing hybridoma cells prepared from immune cells of a primary producer host with a labeled affinity-purified selective antiserum specific for said pathogen, wherein said immune cells have been activated with an affinity-purified antiserum specific for said pathogen; and

selecting antibody-producing cells that bind said detectably labeled antiserum.

2. The method of Claim 1, wherein antibody-producing, non-hybridoma immune cells are incubated and selected.

3. The method of Claim 2, wherein said selective antiserum is obtained from an animal of the species for which protection against said pathogen is desired.

4. The method of Claim 2, wherein said antibody-producing immune cells are incubated with a non-specific antiserum from an animal belonging to the species to which the selective host belongs prior to incubation with said detectably labeled affinity purified selective antiserum.

5. The method of Claim 1, wherein said selective antiserum is labeled with a fluorescent marker.

6. The method of Claim 5, wherein said selecting comprises separating cells with a fluorescent-activated cell sorter.

7. The method of Claim 1, wherein said antibody-producing cells are from an animal from a species different from said selective host and different from a secondary producer host of said activating antiserum.

8. The method of Claim 1, wherein antibody-producing hybridoma cells are incubated and selected.

9. The method of Claim 8, wherein said selective antiserum is obtained from an animal of the species for which protection against said pathogen is desired.

10. The method of Claim 8, wherein said antibody-producing immune cells are incubated with a non-specific antiserum from an animal belonging to the species to which the selective host belongs prior to incubation with said detectably labeled affinity purified selective antiserum.